# FASCICULE DE BREVET EUROPEEN

(11) **EP 3 016 706 B1**
(45) Date de publication et mention de la délivrance du brevet: **17.05.2017**
(21) Numéro de dépôt: 14750549.9
(22) Date de dépôt: 04.07.2014
(51) Int. Cl.: A61M 11/02, A61M 15/00, B05B 11/06

(54) **DISPOSITIF DE DISTRIBUTION DE PRODUIT FLUIDE OU PULVÉRULENT**
SPENDERVORRICHTUNG FÜR FLÜSSIGE ODER PULVERFÖRMIGE PRODUKTE
FLUID OR POWDERY PRODUCT DISPENSING DEVICE

(30) Priorité: 05.07.2013 FR 1356658
(43) Date de publication de la demande: 11.05.2016
(73) Titulaire: Aptar France SAS, 27110 Le Neubourg (FR)
(72) Inventeur: BAILLET, Matthieu, 76000 ROUEN (FR)
(74) Mandataire: CAPRI
(86) Numéro de dépôt international: PCT/FR2014/051727
(87) Numéro de publication internationale: WO 2015/001274

(56) Documents cités:
- EP-A1- 0 575 239
- EP-A2- 1 321 160
- WO-A1-02/45866
- WO-A1-91/12895
- WO-A1-99/46055
- FR-A1- 2 775 963
- US-A- 4 017 007

## Description

La présente invention concerne un dispositif de distribution de produit fluide ou pulvérulent, et plus particulièrement un dispositif pour distribuer unitairement une dose de produit contenu dans un réservoir à l'aide d'un écoulement d'air sous pression.

Le document WO 99/46055 divulgue un tel dispositif dans lequel un élément de fermeture sphérique, qui obture la sortie du réservoir, est expulsé par l'écoulement d'air créé par une chasse d'air. Pour l'utilisation d'un dispositif de distribution de poudre plus particulièrement, la pression d'air nécessaire pour actionner le dispositif devra être suffisamment élevée pour garantir la distribution complète de la dose, ainsi que son fractionnement si cela est nécessaire. Dans le dispositif susmentionné, la pression d'air nécessaire pour actionner le dispositif est déterminée par la résistance donnée par la bille pour être expulsée. Cette résistance est relativement difficile à contrôler et à prédéterminer puisqu'elle est dépendante du frottement entre la bille et son siège cylindrique dans lequel elle est emmanchée pour obturer de manière étanche ledit réservoir. Il peut par conséquent être nécessaire de minimiser l'interférence entre la sphère et son siège cylindrique, ce qui peut évidemment altérer l'efficacité de l'obturation. De plus, il peut être nécessaire de minimiser la profondeur et le positionnement de la sphère dans son siège afin de faciliter son expulsion. Il peut également être nécessaire de fournir une pression d'air relativement élevée qui n'est pas toujours facile à réaliser à l'aide d'un système de pompe ou d'un système de soufflet, notamment lorsque ces chasses d'air sont actionnées manuellement par le patient. De plus, la distribution, c'est à dire l'expulsion de la bille de son siège, peut se produire à des longueurs différentes de la course de la pompe ou du soufflet de la chasse d'air, de sorte que l'instant précis de la distribution du produit ne peut pas être toujours prédéterminé de manière exacte. Enfin, il y a une limitation dans le choix des matériaux pour la sphère et pour son siège.

Le document WO 02/45866 décrit un dispositif dans lequel une bille de fermeture est expulsée mécaniquement par une tige solidaire d'une chasse d'air. Cette mise en oeuvre présente plusieurs inconvénients. Ainsi, l'utilisation d'un élément de fermeture sphérique, tel qu'une bille, réalise l'étanchéité au repos seulement au niveau d'une ligne d'étanchéité, à savoir le diamètre extérieur de la bille. Ceci peut présenter un risque de perte d'étanchéité et impose un dimensionnement précis de la bille et du canal dans lequel ladite bille est coincée. De plus, les billes plastiques devant être rodées, il s'en suit un risque de particules métalliques provenant des outils de rodage des billes.

Les documents WO91/12895 et EP1321160 décrivent d'autres dispositifs de l'art antérieur.

La présente invention a pour but de fournir un dispositif de distribution de produit fluide ou pulvérulent qui ne reproduit pas les inconvénients susmentionnés.

La présente invention a donc pour but de fournir un dispositif de distribution de produit fluide ou pulvérulent, dans lequel l'élément de fermeture assure en toute occasion une fermeture étanche du réservoir au repos.

La présente invention a également pour but de fournir un dispositif de distribution de produit fluide ou pulvérulent du type susmentionné, qui soit simple et peu coûteux à fabriquer et à assembler.

La présente invention a donc pour objet un dispositif de distribution de produit fluide ou pulvérulent tel que défini dans la revendication 1.

Avantageusement, la chasse d'air comporte un piston coulissant dans une chambre d'air.

Ces caractéristiques et avantages et d'autres apparaîtront plus clairement au cours de la description détaillée suivante d'un mode de réalisation avantageux, faite en référence aux dessins joints, donnés à titre d'exemples non limitatifs, et sur lesquels :
- la figure 1 est une vue schématique en section transversale d'un dispositif de distribution de produit fluide ou pulvérulent selon un mode de réalisation avantageux de la présente invention, en position de repos ;
- la figure 2 est une vue similaire à celle de la figure 1, en position d'actionnement ; et
- la figure 3 est une vue schématique découpée en perspective montrant un réservoir de produit fluide ou pulvérulent pour un dispositif de distribution selon un mode de réalisation avantageux de la présente invention.

La présente invention concerne plus particulièrement un dispositif du type de celui divulgué dans le document WO 02/45866. Ce document décrit le fonctionnement général du dispositif.

Il est toutefois entendu que la présente invention ne se limite pas à ce type de dispositif, mais est au contraire applicable à tous les types de dispositifs de distribution de produit fluide et pulvérulents comportant un réservoir obturé par un élément de fermeture, le contenu du réservoir devant être expulsé par un écoulement d'air.

En référence aux figures, il est représenté un mode de réalisation avantageux de l'invention. Le dispositif comporte un réservoir 30 comportant une entrée d'air 31 et une sortie de produit 32. L'entrée d'air 31 du réservoir est reliée à une chasse d'air 20 et la sortie de produit 32 du réservoir est reliée à une sortie de distribution 10 du dispositif. La sortie de produit 32 est obturée par un élément de fermeture 50 qui est emmanché à force dans ladite sortie de produit 32. L'entrée d'air 31 est pourvue d'un organe de retenue de produit 40 qui est adapté à maintenir le produit dans le réservoir avant l'actionnement du dispositif. La chasse d'air 20 est actionnée manuellement par l'utilisateur et est adaptée à créer un écoulement d'air qui va traverser le réservoir 30 pour emmener le produit qu'il contient en direction de la sortie de distribution 10.

Le dispositif comporte un système d'ouverture mécanique 61, 62, qui est solidaire de la chasse d'air 20, c'est à dire qu'il est actionné simultanément à l'actionnement de ladite chasse d'air 20, et qui est adapté à coopérer avec ledit élément de fermeture 50 pour l'expulser mécaniquement de sa position d'obturation lors de l'actionnement du dispositif. Le système d'ouverture mécanique comporte un ensemble de tiges 61, 62, dont une première partie de tige 61 est solidaire de la chasse d'air 20, et une seconde partie de tige 62 est poussée par ladite première partie de tige 61 lorsque le dispositif est actionné. L'ensemble de tiges 61, 62 va en fin de course d'actionnement, c'est à dire en position de distribution, coopérer avec l'élément de fermeture 50 pour l'expulser mécaniquement de sa position d'obturation.

L'organe de retenue de produit 40 est solidaire de la seconde partie de tige 62, et peut être avantageusement réalisé monobloc avec la seconde partie de tige 62. Ainsi, l'organe de retenue de produit 40 peut être réalisé de manière étanche au produit et étanche à l'air, avant l'actionnement du dispositif, la pression d'air créée par la chasse d'air 20 ne pénétrant à l'intérieur du réservoir 30 qu'au moment où ledit organe de retenu 40 est déplacé ensemble avec la seconde partie de tige 62, en étant poussé par la première partie de tige 61.

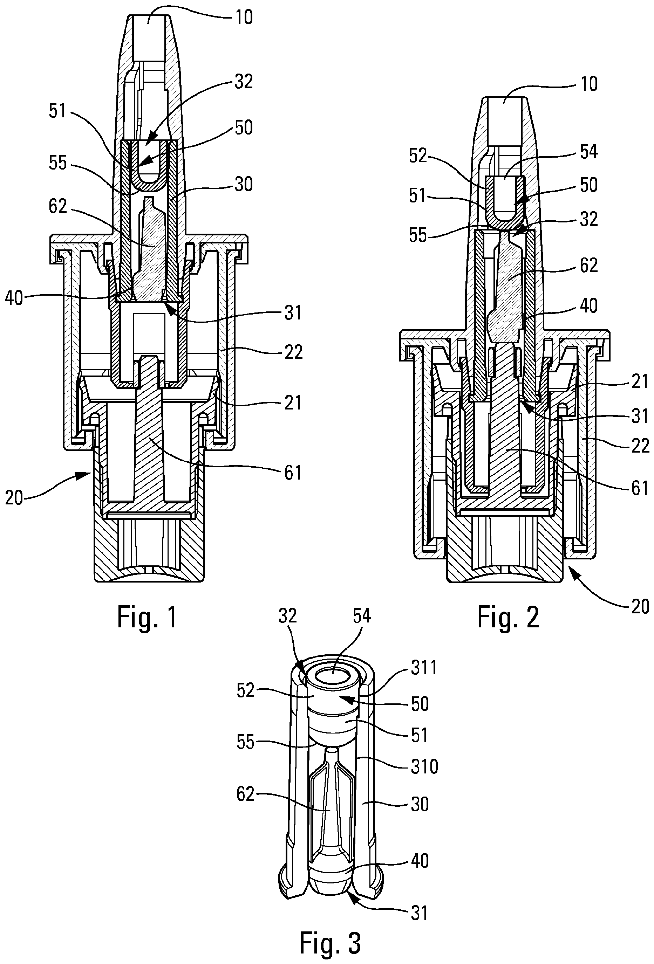

L'organe de retenue de produit 40 est avantageusement réalisé sous la forme d'un disque rigide qui se prolonge à l'intérieur du réservoir par la seconde partie de tige 62. Cette seconde partie de tige 62 est de préférence réalisée avec une partie centrale, qui peut être conique ou d'une forme environ similaire, et plusieurs ailettes latérales, trois dans l'exemple de la figure 3. Ces ailettes assurent notamment un guidage de la seconde partie de tige 62 dans le corps du réservoir lors de l'actionnement. Avantageusement, ledit disque formant l'organe de retenue de poudre est emmanché à force dans le corps du réservoir. En variante, ledit disque peut être fixé, par exemple soudé, au corps du réservoir. Ceci garantit une étanchéité parfaite au repos, et impose de casser ladite soudure au moment de l'actionnement.

La chasse d'air représentée sur les figures 1 et 2 comporte un piston 21 coulissant dans une chambre d'air 22, le piston 21 étant actionné manuellement par l'utilisateur. La présence de la tige, et en particulier de la première partie de tige 61, peut fournir un guidage pour ledit piston 21, ce qui facilite son actionnement, en imposant un déplacement axial dudit piston à l'intérieur de la chambre 22.

Selon l'invention, l'élément de fermeture 50 est non sphérique. Ceci améliore l'étanchéité et évite les particules de métal liées au rodage des billes plastiques. L'élément de fermeture 50 comporte une partie cylindrique 51 qui définit une surface d'étanchéité périphérique avec ladite sortie de produit 32. L'étanchéité est donc formée non pas sur une ligne d'étanchéité, comme avec une bille sphérique, mais sur une surface cylindrique.L'élément de fermeture 50 comporte un manchon cylindrique axial creux. D'un coté axial, qui est celui tourné vers l'intérieur du réservoir 30 en position de fermeture, l'élément de fermeture 50 comporte une partie arrondie 55, notamment semi-sphérique, pour faciliter le passage du produit vers la sortie de distribution 10 lors de l'actionnement. De l'autre côté axial, le manchon creux est ouvert 54. Cette mise en oeuvre donne une certaine souplesse à l'élément de fermeture 50, ce qui facilite sa mise en place et permet en partie de compenser les tolérances dimensionnelles de fabrication pour garantir une fermeture étanche en position de fermeture.

Comme visible sur la figure 3, le coté corps du réservoir 30 définissant la sortie de produit 32 peut comporter une partie de bord axial 311 de diamètre légèrement agrandie par rapport à la partie 310 qui coopère de manière étanche avec l'élément de fermeture 50 en position de fermeture. Le manchon axial creux de l'élément de fermeture 50 peut aussi comporter une partie de bord axial 52 de diamètre extérieur légèrement inférieur par rapport au diamètre extérieur de la partie cylindrique 51 qui coopère de manière étanche avec le corps du réservoir en position de fermeture. Ceci facilite notamment la mise en place de l'élément de fermeture 50.

Bien que décrite principalement en liaison avec un produit pulvérulent, la présente invention s'applique également à la distribution de produits liquides.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Dispositif de distribution de produit fluide ou pulvérulent comportant une sortie de distribution (10), une chasse d'air (20) pour générer un écoulement d'air lors de l'actionnement du dispositif, et au moins un réservoir (30) contenant une dose unique de produit, ledit réservoir (30) comportant une entrée d'air (31) reliée à ladite chasse d'air (20) et une sortie de produit (32) reliée à ladite sortie de distribution (10), ladite entrée d'air (31) comportant un organe de retenue de produit (40) pour maintenir le produit dans le réservoir (30) jusqu'à la distribution du produit, et ladite sortie de produit (32) étant obturée par un élément de fermeture (50) emmanché à force dans la sortie de produit (32) du réservoir (30), ledit dispositif comportant un système d'ouverture mécanique comprenant un ensemble de tiges (61, 62) coopérant en fin de course d'actionnement avec ledit élément de fermeture (50) pour l'expulser mécaniquement de sa position d'obturation lors de l'actionnement du dispositif, ledit ensemble de tiges (61, 62) comportant une première partie de tige (61) solidaire de ladite chasse d'air (20) et une seconde partie de tige (62) solidaire dudit organe de retenue de produit (40), **caractérisé en ce que** l'élément de fermeture (50) comporte un manchon axial cylindrique creux comportant une partie cylindrique (51) qui définit une surface d'étanchéité périphérique avec ladite sortie de produit (32), ledit manchon axial cylindrique creux étant ouvert d'un côté axial (54) et fermé de l'autre côté axial par une partie arrondie (55), notamment semi-sphérique, ladite partie arrondie (55) étant tournée vers l'intérieur du réservoir (30) en position de fermeture.

2. Dispositif selon la revendication 1, dans lequel ladite chasse d'air (20) comporte un piston (21) coulissant dans une chambre d'air (22).

## Patentansprüche

1. Ausgabevorrichtung für ein fluides oder pulverförmiges Produkt, aufweisend eine Ausgabeöffnung (10), einen Blasebalg (20) zum Erzeugen eines Luftstroms bei Betätigung der Vorrichtung und mindestens einen Behälter (30), der eine einzige Produktdosis enthält, wobei der Behälter (30) einen Lufteinlass (31), der mit dem Blasebalg (20) verbunden ist, und einen Produktauslass (32) aufweist, der mit der Ausgabeöffnung (10) verbunden ist, wobei der Lufteinlass (31) eine Produktrückhalteeinrichtung (40) aufweist, um das Produkt bis zur Ausgabe des Produkts in dem Behälter (30) zu halten, und der Produktauslass (32) durch ein Verschlusselement (50) versperrt ist, welches in den Produktauslass (32) des Behälters (30) mit Kraft eingedrückt ist, wobei die Vorrichtung ein mechanisches Öffnungssystem aufweist, welches einen Stangenaufbau (61, 62) enthält, der am Ende des Betätigungshubs mit dem Verschlusselement (50) zusammenwirkt, um es bei der Betätigung der Vorrichtung mechanisch von seiner Absperrposition wegzudrängen, wobei der Stangenaufbau (61, 62) einen ersten Stangenteil (61) in einem Stück mit dem Blasebalg (20) und einen zweiten Stangenteil (62) in einem Stück mit der Produktrückhalteeinrichtung (40) aufweist, **dadurch gekennzeichnet, dass** das Verschlusselement (50) eine axiale zylindrische hohle Muffe aufweist, die einen zylindrischen Teil (51) aufweist, der eine umfängliche Dichtigkeitsfläche mit dem Produktauslass (32) bildet, wobei die axiale zylindrische hohle Muffe auf einer axialen Seite (54) geöffnet und auf der anderen axialen Seite durch einen abgerundeten, insbesondere halbkugelförmigen Teil (55) verschlossen ist, wobei der abgerundete Teil (55) in der Verschlussposition zum Inneren des Behälters (30) weist.

2. Vorrichtung nach Anspruch 1, wobei der Blasebalg (20) einen Kolben (21) aufweist, der in einer Luftkammer (22) gleitet.

## Claims

1. A dispenser device for dispensing a fluid or powder composition, the dispenser device including a dispenser outlet (10), an air expeller (20) for generating a flow of air while the device is being actuated, and at least one reservoir (30) that contains a single dose of a composition, said reservoir (30) including an air inlet (31) that is connected to said air expeller (20), and a composition outlet (32) that is connected to said dispenser outlet (10), said air inlet (31) including a composition retainer member (40) for retaining the composition in the reservoir (30) until the composition is dispensed, and said composition outlet (32) being closed by a closure element (50) that is force fitted in the composition outlet (32) of the reservoir (30), said device including a mechanical opening system that includes a set of rods (61, 62) that, at the end of the actuation stroke, co-operate with said closure element (50) so as to expel it mechanically from its closed position while the device is being actuated, said set of rods (61, 62) comprises a first rod portion (61) that is secured to said air expeller (20), and a second rod portion (62) that is secured to said composition retainer member (40), the device being **characterized in that** the closure element (50) includes a hollow cylindrical axial sleeve comprising a cylindrical portion (51) that defines a peripheral sealing surface with said outlet (32), said hollow cylindrical axial sleeve being open at one axial end (54) and closed at the other axial end by a rounded portion (55), in particular a hemi-spherical portion, said rounded portion (55) facing towards the inside of the reservoir (30) in the closed position.

2. A device according to claim 1, wherein said air expeller (20) includes a piston (21) that slides in an air chamber (22).
